(19) Europäisches Patentamt
European Patent Office
Office européen des brevets

(11) EP 0 988 269 B1

(12) **EUROPÄISCHE PATENTSCHRIFT**

(45) Veröffentlichungstag und Bekanntmachung des Hinweises auf die Patenterteilung:
**04.12.2002 Patentblatt 2002/49**

(21) Anmeldenummer: **98932083.3**

(22) Anmeldetag: **26.05.1998**

(51) Int Cl.[7]: **C07C 45/00**, C07C 47/04

(86) Internationale Anmeldenummer:
**PCT/EP98/03084**

(87) Internationale Veröffentlichungsnummer:
**WO 98/055436 (10.12.1998 Gazette 1998/49)**

(54) **VERFAHREN ZUR HERSTELLUNG VON FORMALDEHYD AUS METHANOL**
METHOD FOR PRODUCING FORMALDEHYDE FROM METHANOL
PROCEDE POUR LA PRODUCTION DE FORMALDEHYDE A PARTIR DE METHANOL

(84) Benannte Vertragsstaaten:
**BE DE FR GB NL**

(30) Priorität: **02.06.1997 DE 19722774**
**30.06.1997 DE 19727519**
**30.06.1997 DE 19727520**
**30.09.1997 DE 19743145**
**31.03.1998 DE 19814285**

(43) Veröffentlichungstag der Anmeldung:
**29.03.2000 Patentblatt 2000/13**

(73) Patentinhaber: **Ticona GmbH**
**65451 Kelsterbach (DE)**

(72) Erfinder:
- **SCHWEERS, Elke**
  **D-69812 Bad Soden (DE)**
- **KAISER, Thomas**
  **D-65779 Kelkheim (DE)**
- **MEISTER, Christine**
  **D-65843 Sulzbach (DE)**
- **ROSENBERG, Michael**
  **D-65527 Niedernhausen (DE)**
- **SCHULZ, Rolf**
  **D-65931 Frankfurt (DE)**

(56) Entgegenhaltungen:
**EP-A- 0 494 350** **EP-A- 0 691 338**
**DE-A- 2 525 174**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 988 269 B1

Printed by Jouve, 75001 PARIS (FR)

## Beschreibung

Mehrere Verfahren zur Herstellung von Formaldehyd aus Methanol sind bekannt (siehe z.B. Ullmann's Encyclopaedia of Industrial Chemistry). Technisch durchgeführt werden überwiegend die Oxidation

$$CH_3OH + \tfrac{1}{2}\, O_2 \rightarrow CH_2O + H_2O$$

an Eisen- und Molybdänoxyd enthaltenden Katalysatoren bei 300°C bis 450°C (Formox Prozeß) und die oxidative Dehydrierung (Silberkontaktverfahren) gemäß:

$$CH_3OH \rightarrow CH_2O + H_2$$

$$H_2 + \tfrac{1}{2}\, O_2 \rightarrow H_2O$$

bei 600°C bis 720°C. Nach beiden Verfahren liegt der Formaldehyd zunächst als wäßrige Lösung vor. Insbesondere bei der Verwendung für die Herstellung von Formaldehyd-Polymeren und -Oligomeren muß der so gewonnene Formaldehyd aufwendig entwässert werden. Ein weiterer Nachteil ist die Bildung der korrosiven, die Polymerisation negativ beeinflussenden Ameisensäure als Nebenprodukt

Durch die Dehydrierung von Methanol können diese Nachteile vermieden und kann im Gegensatz zu oben genannten Verfahren nahezu wasserfreier Formaldehyd direkt gewonnen werden:

$$CH_3OH \xrightarrow{\text{Kat.}} CH_2O + H_2$$

Um ein ökologisches und wirtschaftlich interessantes technisches Verfahren für die Dehydrierung von Methanol zu erreichen, sollten die folgenden Voraussetzungen erfüllt werden: Die stark endotherme Reaktion sollte bei hohen Temperaturen durchgeführf werden, damit hohe Umsätze erreicht werden. Konkurrierende Nebenreaktionen müssen unterdrückt werden, um eine hinreichende Selektivität für Formaldehyd zu erzielen (unkatalysiert beträgt die Selektivität für die Bildung von Formaldehyd bei Umsätzen über 90 % weniger als 10 %). Die Verweilzeiten müssen kurz oder die Abkühlung der Reaktionsprodukte schnell sein, um den Zerfall des thermodynamisch bei den Reaktionsbedingungen nicht stabilen Formaldehyds

$$CH_2O \rightarrow CO + H_2$$

zu verringern.

Verschiedene Verfahren zur Durchführung dieser Reaktion wurden vorgeschlagen; so ist beispielsweise in der DE-A-37 19 055 ein Verfahren zur Herstellung von Formaldehyd aus Methanol durch Dehydrierung in Gegenwart eines Katalysators bei erhöhter Temperatur beschrieben. Die Umsetzung wird in Gegenwart eines mindestens eine Natriumverbindung enthaltenden Katalysators bei einer Temperatur von 300°C bis 800°C durchgeführt.

J. Sauer und G. Emig (Chem. Eng. Technol. 1995, 18, 284-291) gelang es, aus einem $NaAlO_2$ und $LiAlO_2$ enthaltenden Katalysator durch ein reduzierendes Gasgemisch (87%$N_2$ + 13%$H_2$) eine katalytisch aktive Spezies freizusetzen, bei der es sich seiner Vermutung nach um Natrium handelte. Diese Spezies vermag die Dehydrierung von im selben Reaktor stromabwärts zugegebenem, d.h. nicht mit der Katalysatorschüttung in Kontakt gekommenem, Methanol zu Formaldehyd zu katalysieren. Bei Verwendung nicht reduzierender Gase wurde nur geringe katalytische Aktivität beobachtet.

Nach J.Sauer und G.Emig sowie Ergebnissen aus neueren Untersuchungen (siehe z.B. M. Bender et al., Vortrag auf dem XXX. Jahrestreffen deutscher Katalytiker, 21.-23.3.1997) wurden Natriumatome und NaO-Moleküle als in die Gasphase emittierte Verbindungen identifiziert und deren katalytische Aktivität für die Dehydrierung von Methanol in der Gasphase beschrieben.

Das Ausgangsmaterial Methanol wird bei den bekannten Verfahren stets verdünnt mit Stickstoff oder Stickstoff/Wasserstoff-Gemischen umgesetzt.

Obwohl mit den bekannten Verfahren bereits gute Ergebnisse erzielt werden, besteht doch ein breiter Raum für Verbesserungen in technischer und ökonomischer Hinsicht.

In verschiedenen Schriften, wie EP-A 0 130 068, EP-A 0 261 867 und DE-A 25 25 174, wird vorgeschlagen, das bei der Reaktion entstehende Gasgemisch nach Abtrennen des Formaldehyds als Brennstoff zu verwenden.

Es wurde nun überraschend gefunden, daß sich eine in technischer und wirtschaftlicher, insbesondere energetischer Hinsicht stark verbesserte Reaktionsführung erreichen läßt, wenn das neben dem Formaldehyd entstehende Gasgemisch zur Verdünnung des Ausgangsstoffes Methanol eingesetzt wird.

Ein Gegenstand der Erfindung ist daher ein Verfahren zur Herstellung von Formaldehyd aus Methanol durch Dehydrierung in einem Reaktor in Gegenwart eines Katalysators bei einer Temperatur im Bereich von 300 bis 1000°C, dadurch gekennzeichnet, daß man einen Kreisgasstrom, der aus Nebenprodukten der Dehydrierung besteht, durch den Reaktor führt.

Das erfindungsgemäße Verfahren zeichnet sich durch eine ökologisch und ökonomisch günstige Gewinnung von wasserarmen Formaldehyd aus. Durch die Nutzung der wasserstoffreichen Nebenprodukte der Reaktion, d.h. des Produktgases nach Abtrennen des Formaldehyds, zur Verdünnung des Edukts Methanol für die Dehydrierung lassen sich einerseits besonders hohe Ausbeuten erreichen und andererseits wegen der guten Wärmeleitfähigkeit der apparative Aufwand für die Erwärmung der Edukte, den Eintrag der Reaktionswärme sowie die Abkühlung der Produkte minimieren. Durch die weitere mögliche Nutzung von Teilen der Nebenprodukte der Reaktion, d.h. des Produktgases nach Abtrennen des Formaldehyds, als Brennstoff zur Erzeugung der nötigen Reaktionstemperatur für die Dehydrierung, sowie durch eine Wärmerückgewinnung aus den Abgasen kann die Wärme für diesen und weitere Prozeßschritte gewonnen werden. Den Prozeß verlassen dann im wesentlichen nur der Wertstoff Formaldehyd und die Verbrennungsprodukte $CO_2$ und $H_2O$.

Dehydrierung im Sinne der Erfindung bedeutet einen nicht oxidativen Prozeß gemäß der Gleichung:

$$CH_3OH \xrightarrow{Kat} CH_2O + H_2$$

Nebenprodukte im Sinne der Erfindung bezeichnen das nach Abtrennen des Produkts Formaldehyd verbleibende Gasgemisch, welches neben Wasserstoff üblicherweise CO, $CH_4$ und $CO_2$ sowie gegebenenfalls $CH_2O$, MeOH, $H_2O$, $HCOOCH_3$ undloder Rückstände aus der Abtrennung des Formaldehyds enthält und vorzugsweise im wesentlichen aus diesen Gasen besteht. Besonders bevorzugt ist das Verhältnis $H_2$/CO im Kreisgas ≥3.

Fig. 1 gibt einen schematischen Überblick über eine bevorzugte Variante des erfindungsgemäßen Verfahrens.

Aus einem Vorratsgefäß 1 wird Methanol 2 nach Verdünnung mit Kreisgas 8 und Vorwärmen in einem Wärmeaustauscher 3 in den Reaktor 4 geleitet. Nach Durchlaufen des Reaktors 4 wird in einem Wärmetauscher 3' abgekühlt und das Produktgemisch 5 im Separationsgefäß 6 in Formaldehyd 7 und Nebenprodukte 8 (Kreisgas) aufgetrennt. Die Nebenprodukte werden mittels einer Fördereinrichtung 9, beispielsweise einen Ventilator, zumindest teilweise im Kreis in den Reaktor zurückgeführt. Ein Teil der Nebenprodukte kann nach Ausschleusung direkt als Brennmaterial in einer Vorrichtung 10 zur Befeuerung des Reaktionsgefäßes 4 verwendet werden. Bei den Wärmeaustauschern 3, 3' kann es sich um eine Einheit handeln.

Weiterhin Gegenstand der Erfindung ist eine Vorrichtung zur Durchführung des obengenannten Verfahrens enthaltend einen Wärmeaustauscher zum Vorwärmen der Ausgangsstoffe, einen Reaktor zur Durchführung der Dehydrierung, einen Wärmeaustauscher zum Abkühlen des Produktgemisches, ein Separationsgefäß zum Abtrennen des Formaldehyds, sowie Mittel, insbesondere einen Ventilator, zur Rückführung zumindest eines Teils der Nebenprodukte der Reaktion in den Reaktor.

In einer bevorzugten Ausführungsform der erfindungsgemäßen Vorrichtung enthält diese weiterhin Mittel zur Ausschleusung eines weiteren Teils der Nebenprodukte der Dehydrierung, durch die dieser Teil einer Vorrichtung zur Beheizung des Reaktors zugeführt wird und dort als Brennmaterial dient.

Für die Reaktion kann handelsübliches Methanol eingesetzt werden, vorzugsweise sollte es wasserarm sein und keine Stoffe enthalten, die den Katalysator vergiften.

Zur Durchführung der Dehydrierung wird das fluide, vorzugsweise gasförmige Methanol mit gasförmigen Nebenprodukten der Dehydrierung verdünnt.

Der molare Methanolanteil beträgt im allgemeinen 5 bis 90 %, vorzugsweise 10 bis 50 %, besonders bevorzugt 10 bis 40 %. Aus dem Methanolanteil folgt die Menge des benötigten Kreisgases.

Der Druck ist bei dem erfindungsgemäßen Verfahren unkritisch. Die Dehydrierung des Methanols kann bei Unterdruck, Normaldruck oder Überdruck durchgeführt werden. Ein Bereich von etwa 0,1 bis 10 bar, vorzugsweise 0,5 bis 2 bar, ist . besonders geeignet. Bevorzugt ist Normaldruck. Das erfindungsgemäße Verfahren kann diskontinuierlich oder kontinuierlich durchgeführt werden, wobei letzteres bevorzugt ist. Die Temperatur beträgt im allgemeinen 300°C

bis 950°C, bevorzugt 500 bis 900°C, besonders bevorzugt 600 bis 850°C.

Es werden vorzugsweise 0,01 bis 1 kg Methanol pro Stunde und pro Gramm eingesetzten Katalysator umgesetzt. Im Falle eines kontinuierlichen Prozesses muß der Katalysator kontinuierlich oder diskontinuierlich nachgeführt werden. Die Mengen betragen dabei im allgemeinen 10 Milligramm bis 5 Gramm, vorzugsweise 10 mg bis 1 g, besonders bevorzugt 50 bis 1000 mg, ganz besonders bevorzugt 50 bis 500 mg, pro kg umgesetztes Methanol.

Es können beispielsweise die aus der Literatur bekannten Katalysatoren eingesetzt werden, wie sie z.B. in Chem. Eng. Technol. 1994, 17, 34 beschrieben sind.

An Metallen eignen sich beispielsweise Li, Na, K, Cs, Mg, Al, In, Ga, Ag, Cu, Zn, Fe, Ni, Co, Mo, Ti, Pt, und deren Verbindungen. Weiterhin geeignet sind beispielsweise S, Se, Phosphate von Übergangsmetallen, wie V und Fe, und Heteropolysäuren, wie Molybdatophosphorsäure.

Beispiele für konkrete Katalysatoren sind:

- Natrium oder Natriumverbindungen (DE-A-37 19 055 und DE-A-38 11 509)
- Aluminiumoxid, Alkalialuminat und/oder Erdatkaliatuminat (EP-A-04 05 348)
- Silberoxid (JP-A 60/089 441, Dervent Report 85 - 15 68 91/26)
- ein Kupfer, Zink und Schwefel enthaltender Katalysator (DE-A 25 25 174)
- ein Kupfer, Zink und Selen enthaltender Katalysator (US-A 4,054,609)
- ein Zink und/oder Indium enthaltender Katalysator (EP-A 0 130 068)
- Silber (US-A 2,953,602)
- Silber, Kupfer und Silicium (US-A 2,939,883).

Bevorzugt ist die Verwendung von Natrium oder Natriumverbindungen.

Die Anwendungsform eines solchen natriumhaltigen Katalysators kann in weitem Rahmen variieren:
Metallisch, z.B. auch als Legierung mit mindestens einem anderen Legierungsbestandteil, als Verbindung oder Salz, wobei mindestens ein nichtmetallisches Element mit Na gebunden vorliegt (binäre Verbindungen und Salze). Sind mehr als ein Element in der Verbindung gebunden, so liegen binäre, ternäre oder quartäre Verbindungen und Salze vor.

Wird Natrium metallisch eingesetzt, so kann es fest, flüssig oder bevorzugt dampfförmig eingesetzt werden. Bevorzugte Legierungen sind solche mit anderen Alkalimetallen und/oder Erdalkalimetallen, wie Ba, Sr, Ca,Cs, Rb, K oder besonders bevorzugt Li und/oder Magnesium.

Darüber hinaus können auch Legierungen mit B, Al, Si und Sn Verwendung finden. Dies gilt besonders auch für Legierungen, die Verbindungen wie Natriumborid, $NaB_2$, Natriumsilicid, NaSi, oder NaSn enthalten können.

Geeignete binäre Na-Verbindungen und Salze sind beispielsweise Natriumcarbide, wie $Na_2C_2$, $NaC_8$, Natriumhalogenide, wie NaF, Natriumoxide, wie $Na_2O$, Natriumazid, Natriumphosphid, Natriumsulfid, Natriumpolysulfide, bevorzugt auch Natriumhydride, wie NaH.

Geeignete temäre Na-Verbindungen und Salze sind beispielsweise Natriumborate, wie Borax, Natriumphosphate bzw. -hydrogenphosphate, Natriumphosphite, Natrium(meta)silikate und -alumosilikate, wie Wasserglas, $Na_3AlF_6$ (Kryolith), Natrium(hydrogen)sulfat, Natriumsulfit, Natriumnitrit, Natriumnitrat, Natriumamid, Natriumacetylid NaCCH, Natriumcyanid, Natriumrhodanid, Natriumthiomethylat, Natriumthiosulfat, bevorzugt jedoch NaOR, mit R = H, organischer Rest (= Salze organischer Säuren, Alkoholate, Phenolate, Acetylacetonat, Acetessigsäureestersalz, Salze der Salicylsäure oder des Salicylaldehyds), Natriumcarbonat und Natriumhydrogencarbonat und deren Gemische, wie Soda, Thermonatrit, Trona, Pirssonit, Natrocalcit. Generell ist der Einsatz wasserfreier, d.h. getrockneter Salze vorzuziehen.
Besonders bevorzugt sind NaOH, NaOOC-R' (vorzugsweise Formiat, Acetat, Lactat, Oxalat), NaOR= (R= ist ein organisches Radikal mit 1 bis 4 C-Atomen) und Natriumcarbide.
Ganz besonders bevorzugt sind NaOH, Natriumformiat, Natriummethylat, Natriumacetat und Natriumcarbide, wie $Na_2C_2$.

Geeignete quartäre Verbindungen sind z.B. natriumhaltige Alumosilikate, die künstlich hergestellt werden können oder auch vielfältig als natürliche Mineralien und Gesteine (z.B. Natronfeldspat oder Albit und Kalk-Natronfeldspat oder Oligoklas) vorkommen. Sie können zusätzlich durch Ionenaustausch mit Na beladen werden.

Vorteilhaft können auch Doppelsalze vom Typ des Alauns oder Thenardit, Glauberit, Astrakanit, Glaserit, Vanthoffit Verwendung finden.

Die hier genannten Natriumverbindungen und Salze können vorteilhaft auch als Gemische vorliegen. Insbesondere sind durchaus auch Gehalte von < 50%, bevorzugt < 30% von Kationen anderer Alkalimetalle und/oder Erdalkalimetalle, wie Ba, Sr, Ca,Cs, Rb, K oder bevorzugt Li und/oder Magnesium einsetzbar.
Besonders vorteilhaft sind technisch erhältliche, komplexe Gemische, wie Natronkalk, Thomasmehl und Zemente, z. B. Portlandzement, gegebenenfalls nach Anreicherung mit Natrium durch Lagerung in natriumhaltigen Lösungen (NaCl, Meerwasser).

Obengenannte Verbindungen liefern als Katalysatoren Ausbeuten von über 70 % bei Reaktionstemperaturen

von 700 bis 850 °C und geringen Wasserkonzentrationen von weniger als 5 mol-% $H_2O$ pro mol Formaldehyd. Vorteile der tieferen Reaktionstemperatur liegen in dem geringeren energetischen und apparativen Aufwand zur Erwärmung/Abkühlung vor/nach der Reaktion, der geringeren Zerfallsgeschwindigkeit des bei Reaktionbedingungen thermisch instabilen Formaldehyds und den geringeren Anforderungen an die Werkstoffe.

Die obengenannten Stoffe werden im folgenden als Primärkatalysator bezeichnet.

Die Freisetzung der katalytisch aktiven Spezies aus dem Primärkatalysator erfolgt vorzugsweise durch dessen thermische Zersetzung.

Der Primärkatalysator kann beispielsweise als Feststoff, in einem Lösungsmittel gelöst, als Flüssigkeit oder als Schmelze jeweils kontinuierlich oder diskontinuierlich zu- oder nachgeführt werden.

Die Nachführung des Primärkatalysators als Feststoff, z.B. pulverförmig, kömig oder kompaktiert, erfolgt im allgemeinen über eine Feststoffdosierung, z.B. mit Hub- oder Drehkolben, Zellradschleuse, Schnecke oder Schüttelrinne.

Wird der Primärkatalysator gelöst zugegeben, sind besonders Lösungsmittel mit einer chemischen Zusammensetzung geeignet, die nur die im Prozeß schon vorhandenen Elemente (C,H,O) enthalten. Besonders bevorzugt ist MeOH als Lösungsmittel. Die Zugabe erfolgt z.B. über eine Düse, die gekühlt werden kann, um ein Verdampfen des Lösungsmittels, Auskristallisieren oder Ablagerungen des festen Primärkatalysators in der Düse zu vermeiden.

Die Zugabe des Primärkatalysators als Schmelze ist z.B. über eine Düse möglich.
Die Schmelze kann dann direkt im Gasstrom verdampft oder zersetzt werden.

Bei allen Möglichkeiten der Primärkatalysatomachführung geschieht dies vorteilhaft in einer Art und Weise, daß das Material in intensivem Kontakt mit strömendem Gas steht. Dies kann beispielsweise durch Aufbringen des Katalysatormaterials nach den oben beschriebenen Verfahren auf eine geeignete Oberfläche erreicht werden, die vom Gas durch - oder überströmt werden. Es kann sich hierbei um die Oberfläche eines Trägermaterials handeln, das in einem Festbett als Schüttung angeordnet ist. Als Materialien eignen sich z.B. SiC, $SiO_2$ und $Al_2O_3$ in einer geeigneten geometrischen Form, z.B. als Granulat, Pellets oder Kugeln. Die Anordnung geschieht vorzugsweise senkrecht in einem Festbett, vorzugsweise mit Dosierung von oben. Die eingetragene Substanz schlägt sich auf dem Trägermaterial nieder und die katalytisch aktive Spezies geht während des Prozesses in die Gasphase über.

Eine andere Möglichkeit ist die Anordnung des Primärkatalysators in einer Wirbelschicht, durch die der Trägergasstrom geleitet wird. Das Wirbelgut besteht dabei zumindest teilweise aus dem geträgerten oder ungeträgerten Primärkatalysator. Der Verlust an aktiver Substanz kann durch Nachführung von frischem Primärkatalysator ersetzt werden, verbrauchtes Material kann gegebenenfalls abgezogen werden. Dies kann im kontinuierlichen Fall beispielsweise durch eine zirkulierende Wirbelschicht verwirklicht werden.

Eine Nachführung des Primärkatalysators kann auch durch abwechselnde Sekundärkatalysatorerzeugung in verschiedenen Behältern erfolgen, in denen der Primärkatalysator beispielsweise als Festbett oder Wirbelschicht, jeweils geträgert oder ungeträgert, angeordnet sein kann.
Der Vorteil einer Verwendung mehrerer Einheiten zur diskontinuierlichen Katalysatomachführung besteht darin, daß auch solche Primärkatalysatoren eingesetzt werden können, bei denen, z.B. aufgrund von Stoffeigenschaften, wie Schmelzpunkt, Viskosität oder Zersetzungstemperatur, eine kontinuierliche Förderung nicht oder nur mit großem Aufwand möglich wäre.

In einer bevorzugten Variante des erfindungsgemäßen Verfahrens erfolgt die Erzeugung des Sekundärkatalysators räumlich getrennt von der Reaktionszone, in der die eigentliche Dehydrierung stattfindet, und bei einer Temperatur oberhalb der Dehydrierungstemperatur.
Bevorzugt beträgt die Temperaturdifferenz zwischen dem Ort der Katalysatorerzeugung und der Reaktionszone mindestens 20°C, besonders bevorzugt 40 bis 250°C.

Aus den erfindungsgemäßen Primärkatalysatoren werden bei thermischer Behandlung in der Primärkatalysatorzersetzungszone und beim Überströmen mit einem reduzierenden oder auch mit einem nicht reduzierendem Gas, wie molekularem Stickstoff, bei Temperaturen, die nicht gleich der Reaktionstemperatur für die Dehydrierung sondern höher oder niedriger sein können, eine oder mehrere katalytisch aktive Spezies ausgetragen bzw. erzeugt und/oder auf ihm erzeugt (Sekundärkatalysator), die in der Lage sind, die Dehydrierung von Methanol zu katalysieren. Ein solch fluider Katalysator kann über erhebliche Strecken transportiert werden, ohne einen erheblichen Verlust an Wirksamkeit in der Dehydrierung zu erleiden. Diese getrennte Temperatureinstellung erlaubt durch Anpassung an die jeweiligen Bedingungen zur Katalysatorfreisetzungl-verdampfung bzw. Erzeugung einer katalytisch aktiven Spezies (Sekundärkatalysator) einerseits und zur Reaktion andererseits insbesondere die Möglichkeit zur Erniedrigung der Reaktionstemperatur. Damit vermindert sich der Zerfall des unter Reaktionsbedingungen instabilen Formaldehyds durch Folgereaktionen und erhöht sich die Ausbeute.

Bevorzugte Temperaturen zur Erzeugung des Sekundärkatalysators aus dem Primärkatalysator liegen zwischen 300 und 1100°C, besonders bevorzugt sind Temperaturen zwischen 400 und 1000°C.

Weiterhin können die Verweilzeiten im Dehydrierungsreaktor und Behälter zur Primärkatalysatorzugabe bzw. zur Erzeugung des Sekundärkatalysators über die Aufteilung des Trägergasstromes getrennt eingestellt werden. Dadurch wird eine gezielte Beladung des durch die Katalysatorzugabeeinheit geleiteten Gasstromes mit der aktiven Spezies erreicht

Bevorzugte Verweilzeiten zur Erzeugung des Sekundärkatalysators liegen zwischen 0,01 und 60 sec, besonders bevorzugt zwischen 0,05 und 3 sec.

Wird die Erzeugung des Primärkatalysators räumlich getrennt von der Reaktionszone durchgeführt, liegen die Temperaturen in der Reaktionszone im allgemeinen zwischen 200 und 1000°C, vorzugsweise zwischen 300 und 980°C.

Zur Dehydrierung des Methanols sind Verweilzeiten in der Reaktionszone von 0,005 bis 30 sec bevorzugt, besonders bevorzugt sind 0,01 bis 15 sec, ganz besonders bevorzugt 0,05 bis 3 sec.

Geeignete Reaktoren sind dem Fachmann bekannt und geläufig. Grundsätzlich können Reaktortypen und Aufbauten verwendet werden, wie sie aus der Literatur für Dehydrierungsreaktionen bekannt sind. Solche Apparaturen sind beispielsweise in Winnacker/Küchler, Chemische Technologie, 4. Auflage, Kapitel "Technik der Pyrolyse" Hanser Verlag, München 1981-86, beschrieben.

Geeignet sind beispielsweise Rohrreaktoren; geeignete Reaktormaterialien sind beispielsweise keramische Werkstoffe, wie Korund, aber auch aufkohlungs-, temperatur- und zunderbeständige Eisen- und Nickelbasislegierungen, wie Inconel 600® oder Hasteloy®.

Wird der Reaktor durch eine Verbrennungsreaktion beheizt, eignet sich z.B. ein von außen befeuerter Rohrreaktor.

Ebenfalls bevorzugt ist die Beheizung des Reaktors durch Mikrowellen.

Eine weitere bevorzugte Variante des erfindungsgemäßen Verfahrens ist dadurch gekennzeichnet, daß dem Reaktor ein Trägergasstrom zugeführt wird, der eine Temperatur aufweist, die oberhalb der Dehydrierungstemperatur liegt.

Bevorzugt beträgt die Temperaturdifferenz zwischen Trägergasstrom und Dehydrierungstemperatur mindestens 20°C, besonders bevorzugt 40 bis 250°C.

Der überhitzte Gasstrom kann direkt in die Reaktionszone geführt werden oder ganz oder teilweise vorher mit dem Primärkatalysator in Kontakt gebracht werden.

Für den überhitzten Gasstrom liegen die bevorzugten Temperaturen zwischen 600 und 1000°C, besonders bevorzugt zwischen 700 und 900°C. Bevorzugte Temperaturen zur Dehydrierung des Methanols liegen zwischen 500 und 900°C, besonders bevorzugt sind Temperaturen zwischen 600 und 800°C.

Der oder die Trägergasströme können aus einem reduzierenden oder nicht reduzierenden Gas, z.B. $H_2$/CO-Gemischen oder Stickstoff, vorzugsweise aus den Nebenprodukten der Dehydrierung bestehen.

Ein solches Verfahren ist Gegenstand der deutschen Patentanmeldung 197 22 774.0, auf die hiermit ausdrücklich Bezug genommen wird.

Die Abtrennung des Formaldehyds aus dem Reaktionsgemisch kann nach an sich bekannten, dem Fachmann geläufigen Methoden erfolgen, beispielsweise durch Kondensation, Polymerisation oder physikalische oder chemische Ab- oder Adsorption.

Eine technisch erprobte Methode ist die Bildung von Halbacetalen aus Formaldehyd und einem Alkohol. Die Halbacetale werden daran anschließend thermisch gespalten, wobei sehr reiner Formaldehyd-Dampf entsteht. Als Alkohol wird meist Cyclohexanol verwendet, da dessen Siedepunkt genügend weit über der Zersetzungstemperatur des Halbacetals liegt. Die Halbacetale werden üblicherweise in Fallfilm- oder Dünnschichtverdampfem bei Temperaturen von 100 bis 160°C gespalten (siehe z.B. US 2,848,500 vom 19.08.1958 "Preparation of Purified Formaldehyde" und US 2,943,701 vom 05.07.1960 "Process for purification of gaseous formaldehyde", oder JP-A 62/289 540). Die dabei freiwerdenden Formaldehyd-Dämpfe enthalten noch geringe Mengen Verunreinigungen, die meist durch eine Gegenstromwäsche mit Alkohol, wie Cyclohexanolhemiformal, durch Kondensation oder auch durch gezielte Präpolymerisation, entfernt werden.

Besonders bevorzugte Methoden zur Reinigung des erfindungsgemäß hergestellten Formaldehyds sind in den deutschen Patentanmeldungen 19 747 647.3 und 19 748 380.1 beschrieben.

Eine weitere Methode zur Abtrennung von Formaldehyd aus dem Reaktionsgemisch ist die Bildung von Trioxan in einem katalytischen Gasphasenprozeß (siehe z.B. Appl. Catalysis A 1997, 150, 143-151 und EP-A 0 691 338). Trioxan kann dann z.B. auskondensiert werden.

Weitere Verwertungsmöglichkeiten für die Nebenprodukte der Reaktion , insbesondere Wasserstoff, sind beispielsweise die Synthese von Methanol oder die Gewinnung von reinem Wasserstoff, der z.B. durch Membranen abgetrennt werden kann.

So gewonnener Wasserstoff eignet sich beispielsweise zur Synthese von Ammoniak, in Raffinerieprozessen zur Herstellung von Benzin und Krackprodukten der Petrochemie, zur Methanolsynthese, zur Fetthärtung u.a. Hydrierungen, als Reduktionsmittel zur Gewinnung von W, Mo, Co u.a. Metallen, als reduzierendes Schutzgas bei metallurgischen Prozessen, zu autogenen Schweißen und Schneiden, als Brenngas in Mischung mit anderen Gasen (Stadtgas, Wassergas), oder verflüssigt als Treibstoff in Luft- und Raumfahrt.

Der nach dem erfindungsgemäßen Verfahren hergestellte Formaldehyd eignet sich für alle bekannten Einsatzgebiete, beispielsweise Korrosionsschutz, Spiegelherstellung, elektrochemische Beschichtungen, zur Desinfektion und als Konservierungsmittel, ebenso als Zwischenprodukt zur Herstellung von Kunststoffen, beispielsweise Polyoxymethylenen, Polyacetalen, Phenolharzen, Melaminen, Aminoplasten, Polyurethanen und Caseinkunststoffen, 1,4-Butanole, alkoholische Formaldehydlösungen, Methylal, Trimethylolpropan, Neopentylglykol, Pentaerythrit und Trioxan, zur Hestellung von Farbstoffen, wie Fuchsin, Acridin, zur Herstellung von Düngemitteln sowie zur Behandlung von Saatgut.

Die Erfindung betrifft auch derartig hergestellte Kunststoffe, wie Polyoxymethylen und Polyacetale, Trioxan, Farbstoffe, Düngemittel und Saatgut.

Weiterhin Gegenstand der Erfindung ist ein Verfahren zur Herstellung von Trioxan, dadurch gekennzeichnet, daß man

1. Methanol durch Dehydrierung in einem Reaktor in Gegenwart eines Katalysators bei einer Temperatur im Bereich von 300 bis 1000°C zu Formaldehyd umsetzt, wobei man einen Kreisgasstrom, der aus Nebenprodukten der Dehydrierung besteht, durch den Reaktor führt, und
2. den so hergestellten Formaldehyd gegebenenfalls reinigt und zu Trioxan trimerisiert.

Einzelheiten der Herstellung von Trioxan sind dem Fachmann bekannt und geläufig. Sie sind z.B. in Kirk u. Othmer, Encyclopedia of Chemical Technology, 2. Aufl., Band 10, S. 83, 89, New York Interscience 1963-1972, beschrieben.

Gegenstand der Erfindung ist ebenso ein Verfahren zur Herstellung von Polyoxymethylen, dadurch gekennzeichnet, daß man

1. Methanol durch Dehydrierung in einem Reaktor in Gegenwart eines Katalysators bei einer Temperatur im Bereich von 300 bis 1000°C zu Formaldehyd umsetzt, wobei man einen Kreisgasstrom, der aus Nebenprodukten der Dehydrierung besteht, durch den Reaktor führt, und
2. den so gewonnenen Formaldehyd gegebenenfalls reinigt,
3. den Formaldehyd polymerisiert,
4. die Endgruppen des so hergestellten Polymers absättigt (Capping) und
5. gegebenenfalls das Polymer in der Schmelze homogenisiert und/oder mit geeigneten Zusätzen versieht.

Die Herstellung von Polyoxymethylen aus Formaldehyd ist dem Fachmann bekannt und geläufig. Einzelheiten finden sich z.B. in Ullmann's Encyclopedia of Industrial chemistry, Bd. 21, 5. Aufl., Weinheim 1992, und der dort zitierten Literatur.

Ein weiterer Gegenstand der Erfindung ist ein Verfahren zur Herstellung Polyoxymethylen Copolymeren, dadurch gekennzeichnet, daß man

1. Methanol durch Dehydrierung in einem Reaktor in Gegenwart eines Katalysators bei einer Temperatur im Bereich von 300 bis 1000°C zu Formaldehyd umsetzt, wobei man einen Kreisgasstrom, der aus Nebenprodukten der Dehydrierung besteht, durch den Reaktor führt, und
2. den so gewonnenen Formaldehyd zu Trioxan trimerisiert,
3. gegebenenfalls das Trioxan reinigt,
4. das Trioxan mit cyclischen Ethern oder cyclischen Acetalen copolymerisiert,
5. gegebenenfalls instabile Endgruppen entfernt und
6. das so hergestellte Polymer gegebenenfalls in der Schmelze homogenisiert und/oder mit geeigneten Zusatzstoffen versetzt.

Ein weiterer Gegenstand der Erfindung ist ein Verfahren zur Herstellung Polyoxymethylen Copolymeren, dadurch gekennzeichnet, daß man

1. Methanol durch Dehydrierung in einem Reaktor in Gegenwart eines Katalysators bei einer Temperatur im Bereich von 300 bis 1000°C zu Formaldehyd umsetzt, wobei man einen Kreisgasstrom, der aus Nebenprodukten der Dehydrierung besteht, durch den Reaktor führt, und
2. den so gewonnenen Formaldehyd gegebenenfalls reinigt,
3. den Formaldehyd mit cyclischen Ethern oder cyclischen Acetalen copolymerisiert,
4. gegebenenfalls instabile Endgruppen entfernt und
5. das so hergestellte Polymer gegebenenfalls in der Schmelze homogenisiert und/oder mit geeigneten Zusatzstoffen versetzt.

Die Herstellung von Polyoxymethylen-Copolymeren ist dem Fachmann bekannt und geläufig. Einzelheiten finden sich beispielsweise in Ullmann's Encyclopedia of Industrial Chemistry, Bd. 21, 5. Aufl., Weinheim 1992 und der dort zitierten Literatur, sowie in russischen Schriften SU 436067, 740715 und SU 72-1755156, 720303.

Auf den Inhalt der prioritätsbegründenden deutschen Patentanmeldungen 197 22 774.0, 197 27 519.2, 197 27 520.6 und 197 43 145.3 sowie auf die Zusammenfassung der vorliegenden Anmeldung wird ausdrücklich Bezug genommen.

Die Erfindung wird durch die Beispiele näher erläutert, ohne sie dadurch beschränken zu wollen.

Beispiele

Die angegebenen Meßgrößen werden wie folgt berechnet:

$$\text{Umsatz (in \%)} = \frac{\text{umgesetztes Methanol (mol)}}{\text{zudosiertes Methanol (mol)}} \cdot 100$$

$$\text{Ausbeute (in \%)} = \frac{\text{gebildeter Formaldehyd (mol)}}{\text{zudosiertes Methanol (mol)}} \cdot 100$$

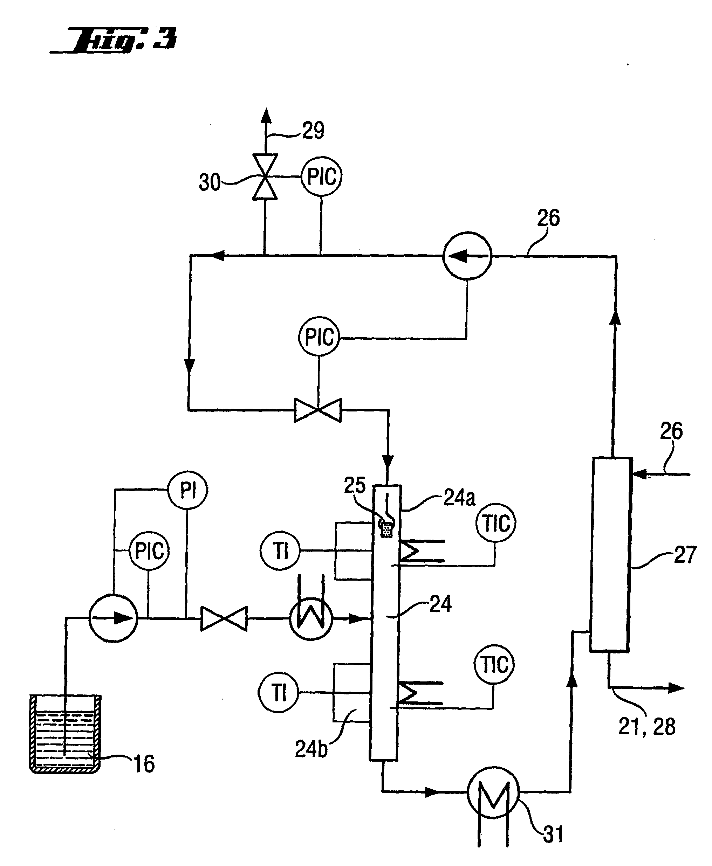

Im folgenden werden Experimente zur Dehydrierung von Methanol beschrieben, die in einem mikrowellenbeheizten und in einem elektrisch beheizten Laborreaktor durchgeführt wurden. Figur 2 zeigt schematisch anhand eines Fließbildes den Aufbau des mikrowellenbeheizten Laborreaktors, Figur 3 den des elektrischbeheizten.

A Mikrowellenbeheizter Reaktor

Der eigentliche Reaktionsraum ist der Lückenraum in einer Schüttung 11. Die Schüttung 11 besteht aus SiC-Kugeln mit wenigen Millimetern Durchmesser und befindet sich in einem Quarzreaktor 12. Zur Beheizung ist der Reaktor 12 in einem Mikrowellenapplikator 13 aufgebaut, wo durch die Bestrahlung in den SiC-Kugeln Wärme freigesetzt wird. Durch diese Art der Reaktorheizung kann eine deutlich homogenere Temperaturverteilung als z.B. in von außen beheizten Rohren erreicht werden. Die Temperatur wird in der Schüttung gemessen und mittels Regelung (TIC) der Strahlungsleistung eingestellt.

Ein Trägergas 15 (vorzugsweise Stickstoff) durchströmt die Schüttung 11 von unten nach oben. Methanol 16 wird durch ein senkrecht angeordnetes Rohr 17 bis auf etwa die halbe Höhe der Schüttung 11 geführt und gelangt dort durch eine Fritte 18 in die Schüttung 11, wo es sich mit dem Trägergas 15 vermischt.

Primärkatalysatoren 19 in Form von Körnern sind in das untere Viertel der Schüttung 11 eingebracht. Außer dem Methanol 16 wird dem Reaktor 12 entweder das Trägergas 15 zugeführt oder die Reaktionsprodukte, nach Abtrennung des Formaldehyds 21 in einer Kühlfalle 22, als Kreisgas 20 in den Reaktor 12 zurückgeführt. Überschüssiges Reaktion- und/oder Trägergas 15 kann über ein Ventil 23 druckgesteuert ausgeschleußt werden.

Der Gesamtvolumenstrom liegt zwischen 20 l/h und 500 l/h, die Verweilzeit innerhalb der Schüttung variert zwischen 0,02 und 1 s und der Methanolanteil liegt zwischen 5 und 50 mol-%. Als Feststoff werden verschiedene Alkaliverbindungen eingesetzt. Das Reaktionsprodukt wird mittels eines Gaschromatographen analysiert.

Tabelle 1

| Umsatz und Formaldehydausbeute derPyrolyse von Methanol (Verweilzeit bei Reaktionstemperatur ca. 0,2s, Eingangskonzentration Methanol ca. 10 mol-%, Katalysatormenge entsprechend ca. 0,5 g Alkali) | | | | | |
|---|---|---|---|---|---|
| Beispiel/ Vergleichsbeispiel | Feststoff (Katalysator) | Temperatur in der Schüttung | Umsatz von Methanol | Ausbeute in N2 | Ausbeute in Kreisgas |
| Bsp. 1 | ohne | ca. 910°C | 60 % | | 12 % |
| VB 1 | ohne | ca. 920°C | 70 % | 11 % | |
| Bsp. 2 | $Na_2CO_3$ | ca. 880°C | 93 % | | 67 % |
| VB 2 | $Na_2CO_3$ | ca. 880°C | 92% | 63% | |
| Bsp. 3 | NaOH | - | 80 % | | 54 % |
| Bsp. 4 | $NaCHO_2$ | ca. 790°C | 91% | | 65 % |
| VB 3 | $NaCHO_2$ | ca. 820°C | 83% | 52 % | |

Tabelle 1 (fortgesetzt)

| Umsatz und Formaldehydausbeute derPyrolyse von Methanol (Verweilzeit bei Reaktionstemperatur ca. 0,2s, Eingangskonzentration Methanol ca. 10 mol-%, Katalysatormenge entsprechend ca. 0,5 g Alkali) | | | | | |
|---|---|---|---|---|---|
| Beispiel/ Vergleichsbeispiel | Feststoff (Katalysator) | Temperatur in der Schüttung | Umsatz von Methanol | Ausbeute in N2 | Ausbeute in Kreisgas |
| Bsp. 5 | $NaC_2H_3O_2$ | ca. 780°C | 92% | | 63 % |
| VB 4 | $NaC_2H_3O_2$ | ca. 760°C | 84% | 55 % | |
| Bsp. 6 | $LiOCH_3$ | ca. 780°C | 91% | | 39 % |
| VB 5 | $LiOCH_3$ | ca. 795°C | 95% | 32 % | |
| Bsp. 7 | $Cs_2CO_3$ | ca. 865°C | 90% | | 26 % |
| VB 6 | $Cs_2CO_3$ | ca. 900°C | 90% | 22 % | |
| Bsp. 8 | $KOCH_3$ | ca. 845°C | 88% | | 27 % |
| VB 7 | $KOCH_3$ | ca. 840°C | 88% | 20 % | |
| Bsp. 9 | $Na_2C_2$ | ca. 765°C | 77% | | 58 % |
| Bsp. 10 | $Na_2C_2$ | ca. 775°C | 89% | | 58 % |
| VB 8 | $Na_2C_2$ | ca. 805°C | 80% | 53 % | |
| Bsp. 11 | $Na_2(COO)_2$ | ca. 785°C | 87% | | 51% |
| Bsp. 12 | $NaOCH_3$ | ca. 760°C | 91 % | | 70 % |
| VB 9 | $NaOCH_3$ | ca. 760°C | 89% | 62 % | |

Tabelle 2

| Umsatz und Formaldehydausbeute der Pyrolyse von Methanol (Verweilzeit bei Reaktionstemperatur ca. 0,1 s, Eingangskonzentration Methanol ca. 10 mol-%, Katalysatormenge entsprechend ca. 0,5 g Alkali) | | | | | |
|---|---|---|---|---|---|
| Beispiel/ Vergleichsbeispiel | Feststoff (Katalysator) | Temperatur in der Schüttung | Umsatz von Methanol | Ausbeute in N2 | Ausbeute in Kreisgas |
| VB10 | $NaC_2H_3O_2$ | ca. 785°C | 92 % | | 76 % |
| Bsp. 13 | $NaC_2H_3O_2$ | ca. 805°C | 92 % | 69 % | |
| Bsp. 14 | $Na_2C_2$ | ca. 780°C | 89 % | | 67 % |
| Bsp. 15 | $Na_2C_2$ | ca. 765°C | 83 % | | 68 % |
| Bsp. 16 | $Na_2(COO)_2$ | ca. 775°C | 94 % | | 68 % |
| VB 11 | $Na_2(COO)_2$ | ca. 765°C | 81 % | 62 % | |

B. Elektrisch beheizter Reaktor

Die Dehydrierung des Methanols 16 wird in einem mittels elektrischen Laboröfen beheizten Rohrreaktor 24 durchgeführt (siehe. Fig. 3). In einem Rohr im ersten Ofen 24a (700 - 1000°C) wird der Katalysator 25 (0,1 - 5g) vorgelegt und vom Kreisgas 26 überströmt. Stromabwärts von diesem wird das vorgewärmte Methanol 16 (20 - 500 g/h, 400 - 800°C) zugeführt. Dann folgt die eigentliche Reaktionszone 24b (Rohr; Länge von 20 - 50 cm, Innendurchmesser 7 - 20 mm) in einem zweiten Ofen (700- 1000°C). Die Reaktionsprodukte werden sofort nach dem Reaktor auf ca. 150°C abgekühlt 31. In einer Kolonne 27 werden die Reaktionsprodukte mit Alkohol 28 (z.B. Cyclohexanol bei 20 - 80 °C) gewaschen, um den Formaldehyd 21 auszutragen 21,28. Von den gasförmingen Produkten nach der Wäsche wird ein Überschuß 29 mittels eines Ventils 30 druckgesteuert (PIC) ausgeschleust und der Rest als Kreisgas 26 (100 - 2000 l/h) zurückgeführt und im ersten Ofen 24a vorgewärmt.

Bei der Einstellung:

80 g/h Methanol

400 l/h Kreisgas
1 g Natriummethylat als Katalysator
werden 54 g/h Formaldehyd gewonnen.

**Patentansprüche**

1. Verfahren zur Herstellung von Formaldehyd aus Methanol durch Dehydrierung in einem Reaktor in Gegenwart eines Katalysators bei einer Temperatur im Bereich von 300 bis 1000°C, **dadurch gekennzeichnet, daß** man einen Kreisgasstrom, der aus Nebenprodukten der Dehydrierung besteht, durch den Reaktor führt.

2. Verfahren gemäß Anspruch 1, **dadurch gekennzeichnet, daß** der Kreisgasstrom im wesentlichen aus $H_2$ und CO besteht.

3. Verfahren gemäß Anspruch 1 und/oder 2, **dadurch gekennzeichnet, daß** das molare Verhältnis $H_2/CO$ im Kreisgas $\geq 3$ ist.

4. Verfahren gemäß einem oder mehreren der Ansprüche 1 bis 3, **dadurch gekennzeichnet, daß** der Reaktor ein von außen befeuerter Rohrreaktor ist.

5. Verfahren gemäß einem oder mehreren der Ansprüche 1 bis 3, **dadurch gekennzeichnet, daß** der Reaktor durch Mikrowellen beheizt wird.

6. Verfahren gemäß einem oder mehreren der Ansprüche 1 bis 4, **dadurch gekennzeichnet, daß** man einen weiteren Teil der Nebenprodukte der Dehydrierung als Brennstoff zur Beheizung des Reaktors verwendet.

7. Verfahren gemäß einem oder mehreren der Ansprüche 1 bis 6, **dadurch gekennzeichnet, daß** man als Katalysator Natrium oder eine Natriumverbindung verwendet, die neben Na lediglich Elemente aus der Gruppe C, H und O enthält.

8. Vorrichtung zur Durchführung eines Verfahrens gemäß einem oder mehreren der Ansprüche 1 bis 7, enthaltend einen Wärmeaustauscher, zum Vorwärmen der Ausgangsstoffe, einen Reaktor zur Durchführung der Dehydrierung, einen Wärmeaustauscher zum Abkühlen des Produktgemisches, ein Separationsgefäß zum Abtrennen des Formaldehyds, sowie Mittel, insbesondere einen Ventilator, zur Rückführung zumindest eines Teil der Nebenprodukte der Dehydrierung (Kreisgas) in den Reaktor.

9. Vorrichtung gemäß Anspruch 8, **dadurch gekennzeichnet, daß** sie Mittel zur Ausschleusung eines weiteren Teils der Nebenprodukte der Dehydrierung enthält, durch die dieser Teil einer Vorrichtung zur Beheizung der Reaktion zugeührt wird und dort als Brennmaterial dient.

10. Verfahren nach einem oder mehreren der Ansprüche 1 bis 7, **dadurch gekennzeichnet, daß** man Teile des als Nebenprodukt anfallenden Wasserstoffs abtrennt und einer weiteren Verwertung zuführt.

11. Verfahren zur Herstellung von Trioxan, **dadurch gekennzeichnet, daß** man

    - Methanol durch Dehydrierung in einem Reaktor in Gegenwart eines Katalysators bei einer Temperatur im Bereich von 300 bis 1000°C zu Formaldehyd umsetzt, wobei man einen Kreisgasstrom, der aus Nebenprodukten der Dehydrierung besteht, durch den Reaktor führt, und
    - den so hergestellten Formaldehyd zu Trioxan trimerisiert.

12. Verfahren zur Herstellung von Polyoxymethylen, **dadurch gekennzeichnet, daß** man

    - Methanol durch Dehydrierung in einem Reaktor in Gegenwart eines Katalysators bei einer Temperatur im Bereich von 300 bis 1000°C zu Formaldehyd umsetzt, wobei man einen Kreisgasstrom, der aus Nebenprodukten der Dehydrierung besteht, durch den Reaktor führt, und
    - den so gewonnen Formaldehyd gegebenenfalls reinigt,
    - den Formaldehyd polymerisiert,
    - die Endgruppen des so hergestellten Polymers absättigt (Capping) und

- gegebenenfalls das Polymer in der Schmelze homogenisiert und/oder mit geeigneten Zusätzen versieht.

**13.** Verfahren zur Herstellung Polyoxymethylen-Copolymeren, **dadurch gekennzeichnet, daß** man

- Methanol durch Dehydrierung in einem Reaktor in Gegenwart eines Katalysators bei einer Temperatur im Bereich von 300 bis 1000°C zu Formaldehyd umsetzt, wobei man einen Kreisgasstrom, der aus Nebenprodukten der Dehydrierung besteht, durch den Reaktor führt, und
- den so gewonnenen Formaldehyd zu Trioxan trimerisiert,
- gegebenenfalls das Trioxan reinigt,
- das Trioxan mit cyclischen Ethern oder cyclischen Acetalen copolymerisiert,
- gegebenenfalls instabile Endgruppen entfernt und
- das so hergestellte Polymer gegebenenfalls in der Schmelze homogenisiert und/oder mit geeigneten Zusatzstoffen versetzt.

**14.** Verfahren zur Herstellung Polyoxymethylen Copolymeren, **dadurch gekennzeichnet, daß** man

- Methanol durch Dehydrierung in einem Reaktor in Gegenwart eines Katalysators bei einer Temperatur im Bereich von 300 bis 1000°C zu Formaldehyd umsetzt, wobei man einen Kreisgasstrom, der aus Nebenprodukten der Dehydrierung besteht, durch den Reaktor führt, und
- den so gewonnenen Formaldehyd gegebenenfalls reinigt,
- den Formaldehyd mit cyclischen Ethern oder cyclischen Acetalen copolymerisiert,
- gegebenenfalls instabile Endgruppen entfernt und
- das so hergestellte Polymer gegebenenfalls in der Schmelze homogenisiert und/oder mit geeigneten Zusatzstoffen versetzt.

## Claims

**1.** A process for preparing formaldehyde from methanol by dehydrogenation in a reactor in the presence of a catalyst at a temperature in the range from 300 to 1000°C, wherein a circulating gas stream comprising by-products of the dehydrogenation is passed through the reactor.

**2.** The process as claimed in claim 1, wherein the circulating gas stream consists essentially of $H_2$ and CO.

**3.** The process as claimed in claim 1 and/or 2, wherein the molar ratio $H_2/CO$ in the circulating gas is $\geq 3$.

**4.** The process as claimed in one or more of claims 1 to 3, wherein the reactor is an externally fired tube reactor.

**5.** The process as claimed in one or more of claims 1 to 3, wherein the reactor is heated by means of microwaves.

**6.** The process as claimed in one or more of claims 1 to 4, wherein a further part of the by-products of the dehydrogenation is used as fuel for heating the reactor.

**7.** The process as claimed in one or more of claims 1 to 6, wherein the catalyst used is sodium or a sodium compound which contains, apart from Na, only elements selected from the group consisting of C, H and O.

**8.** An apparatus for carrying out a process as claimed in one or more of claims 1 to 7, comprising a heat exchanger for preheating the starting materials, a reactor for carrying out the dehydrogenation, a heat exchanger for cooling the product mixture, a separation vessel for separating off the formaldehyde and also means, in particular a fan, for recirculating at least part of the by-products of the dehydrogenation (circulating gas) to the reactor.

**9.** An apparatus as claimed in claim 8 which comprises means for discharging a further part of the by-products of the dehydrogenation and feeding this part to an apparatus for heating the reaction in which latter apparatus it serves as fuel.

**10.** The process as claimed in one or more of claims 1 to 7, wherein part of the hydrogen obtained as byproduct is separated off and passed to a further use.

**11.** A process for preparing trioxane, which comprises

- converting methanol into formaldehyde by dehydrogenation in a reactor in the presence of a catalyst at a temperature in the range from 300 to 1000°C, where a circulating gas stream comprising by-products of the dehydrogenation is passed through the reactor, and
- trimerizing the formaldehyde prepared in this way to give trioxane.

**12.** A process for preparing polyoxymethylene, which comprises

- converting methanol into formaldehyde by dehydrogenation in a reactor in the presence of a catalyst at a temperature in the range from 300 to 1000°C, where a circulating gas stream comprising by-products of the dehydrogenation is passed through the reactor, and
- if desired, purifying the formaldehyde obtained in this way,
- polymerizing the formaldehyde,
- capping the end groups of the polymer prepared in this way and
- if desired, homogenizing the polymer in the melt and/or providing it with suitable additives.

**13.** A process for preparing polyoxymethylene copolymers, which comprises

- converting methanol into formaldehyde by dehydrogenation in a reactor in the presence of a catalyst at a temperature in the range from 300 to 1000°C, where a circulating gas stream comprising by-products of the dehydrogenation is passed through the reactor, and
- trimerizing the formaldehyde obtained in this way to give trioxane,
- if desired, purifying the trioxane,
- copolymerizing the trioxane with cyclic ethers or cyclic acetals,
- if desired, removing unstable end groups and
- if desired, homogenizing the polymer prepared in this way in the melt and/or admixing it with suitable additives.

**14.** A process for preparing polyoxymethylene copolymers, which comprises

- converting methanol into formaldehyde by dehydrogenation in a reactor in the presence of a catalyst at a temperature in the range from 300 to 1000°C, where a circulating gas stream comprising by-products of the dehydrogenation is passed through the reactor, and
- if desired, purifying the formaldehyde obtained in this way,
- copolymerizing the formaldehyde with cyclic ethers or cyclic acetals,
- if desired, removing unstable end groups and
- if desired, homogenizing the polymer prepared in this way in the melt and/or admixing it with suitable additives.

## Revendications

**1.** Procédé de fabrication de formaldéhyde à partir du méthanol par déshydrogénation dans un réacteur en présence d'un catalyseur à une température dans le domaine de 300 à 1000°C, **caractérisé en ce que** l'on conduit à travers le réacteur un courant gazeux de circulation, qui se compose des produits secondaires de la déshydrogénation.

**2.** Procédé selon la revendication 1, **caractérisé en ce que** le courant gazeux de circulation se compose essentiellement de $H_2$ et de CO.

**3.** Procédé selon la revendication 1 et/ou la revendication 2, **caractérisé en ce que** le rapport molaire $H_2$/CO dans le gaz de circulation est égal ou supérieur à 3.

**4.** Procédé selon l'une quelconque des revendications 1 à 3, **caractérisé en ce que** le réacteur est un réacteur tubulaire chauffé de l'extérieur.

**5.** Procédé selon l'une quelconque des revendications 1 à 3, **caractérisé en ce que** le réacteur est chauffé par microondes.

**6.** Procédé selon l'une quelconque des revendications 1 à 4, **caractérisé en ce que** l'on utilise, en tant que combustible, en vue du chauffage du réacteur, une partie supplémentaire des produits secondaires de la déshydrogénation.

**7.** Procédé selon l'une quelconque des revendications 1 à 6, **caractérisé en ce que** l'on utilise, en tant que catalyseur, du sodium ou un composé du sodium, qui contient, outre le Na, simplement des éléments du groupe C, H et O.

**8.** Dispositif en vue de l'exécution d'un procédé selon l'une quelconque des revendications 1 à 7, contenant un échangeur thermique, en vue du chauffage préalable des substances de départ, un réacteur en vue de l'exécution de la déshydrogénation, un échangeur thermique en vue du refroidissement du mélange de produits, un récipient de séparation en vue de la séparation du formaldéhyde ainsi que des moyens, en particulier un ventilateur en vue du retour d'au moins une partie des produits secondaires de la déshydrogénation (gaz de circulation) dans le réacteur.

**9.** Dispositif selon la revendication 8, **caractérisé en ce qu'**il contient des moyens en vue de l'éclusage au dehors d'une partie supplémentaire des produits secondaires de la déshydrogénation, à travers laquelle cette partie est acheminée à un dispositif en vue du chauffage de la réaction et y sert. de matériau combustible.

**10.** Procédé selon l'une quelconque des revendications 1 à 7, **caractérisé en ce que** l'on sépare des parties de l'hydrogène qui se forme en tant que produit secondaire et **en ce que** l'on achemine celles-ci à une récupération supplémentaire.

**11.** Procédé de fabrication de trioxanne, **caractérisé en ce que**

- l'on fait réagir le méthanol par déshydrogénation dans un réacteur en présence d'un catalyseur à une température dans le domaine de 300 à 1000EC pour former le formaldéhyde, un courant gazeux de circulation, qui se compose des produits secondaires de la déshydrogénation, étant acheminé à travers
- le réacteur et le formaldéhyde ainsi fabriqué étant trimérisé pour former du trioxanne.

**12.** Procédé de fabrication de polyoxyméthylène, **caractérisé**

- **en ce que** l'on fait réagir le méthanol par déshydrogénation dans un réacteur en présence d'un catalyseur à une température dans le domaine de 300 à 1000EC pour former le formaldéhyde, un courant gazeux de circulation qui se compose des produits secondaires de la déshydrogénation, étant acheminé à travers le réacteur et
- **en ce que** l'on nettoie, le cas échéant, le formaldéhyde ainsi obtenu,
- **en ce que** l'on procède à la polymérisation du formaldéhyde,
- **en ce que** l'on procède à la saturation des groupements terminaux du polymère ainsi fabriqué (capping) et
- **en ce que** l'on procède, le cas échéant, à l'homogénéisation du polymère dans la masse fondue et/ou en ce qu'on le pourvoit d'additifs appropriés.

**13.** Procédé de fabrication de copolymères de polyoxyméthylène, **caractérisé**

- **en ce que** l'on fait réagir le méthanol par déshydrogénation dans un réacteur en présence d'un catalyseur à une température dans le domaine de 300 à 1000EC pour former le formaldéhyde, un courant gazeux de circulation qui se compose des produits secondaires de la déshydrogénation, étant acheminé à travers le réacteur et
- **en ce que** l'on trimérise le formaldéhyde ainsi obtenu pour former du trioxanne,
- **en ce que** l'on nettoie, le cas échéant, le trioxanne ainsi obtenu,
- **en ce que** l'on procède à la copolymérisation du trioxanne à l'aide d'éthers cycliques ou d'acétals cycliques,
- en ce l'on procède à l'élimination des groupements terminaux, le cas échéant, instables et
- **en ce que** l'on procède à l'homogénéisation du polymère ainsi fabriqué, le cas échéant, dans la masse fondue et/ou que l'on fait réagir celui-ci avec des substances additives appropriées.

**14.** Procédé de fabrication de copolymères de polyoxyméthylène, **caractérisé**

- **en ce que** l'on fait réagir le méthanol par déshydrogénation dans un réacteur en présence d'un catalyseur à une température dans le domaine de 300 à 1000EC pour former le formaldéhyde, un courant gazeux de circulation qui se compose des produits secondaires de la déshydrogénation, étant acheminé à travers le

réacteur et

- **en ce que** l'on nettoie, le cas échéant, le formaldéhyde ainsi obtenu,
- **en ce que** l'on procède à la copolymérisation du formaldéhyde à l'aide d'éthers cycliques ou d'acétals cycliques,
- en ce l'on procède à l'élimination des groupements terminaux, le cas échéant, instables et
- **en ce que** l'on procède à l'homogénéisation du polymère ainsi fabriqué, le cas échéant, dans la masse fondue et/ou en ce que l'on fait réagir celui-ci avec des substances additives appropriées.

Fig. 1

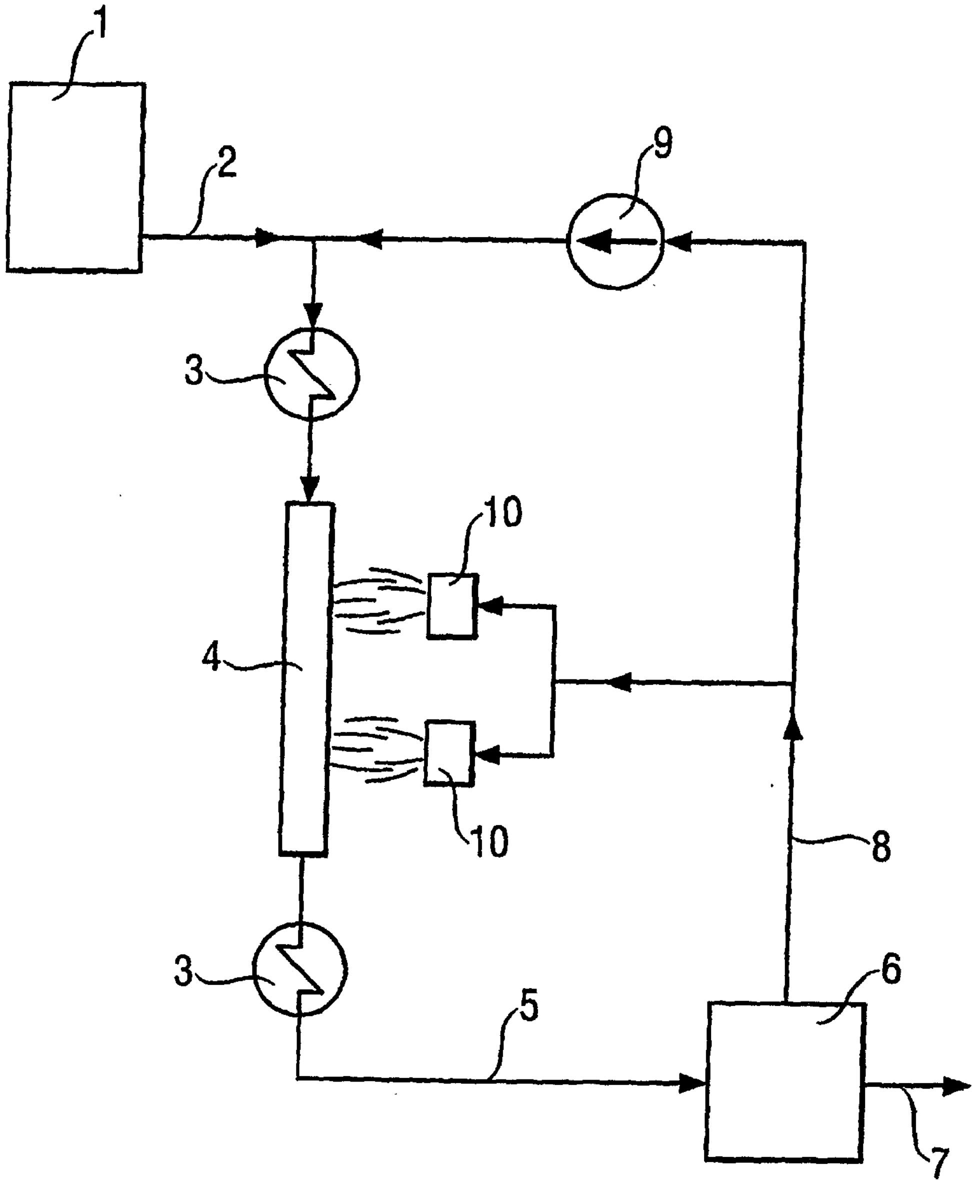

Fig. 2

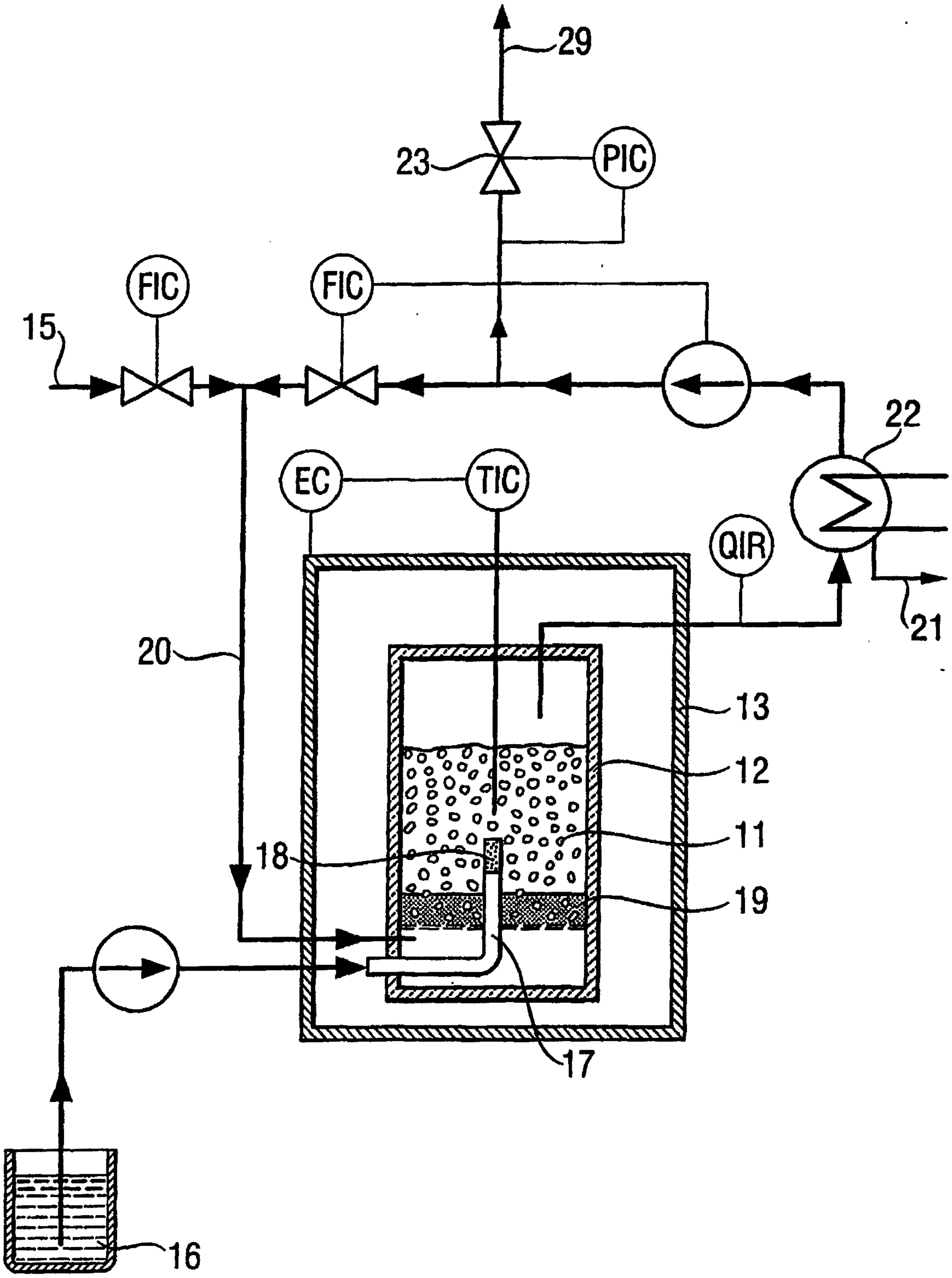

Fig. 3